# EUROPEAN PATENT APPLICATION

(11) **EP 4 628 020 A1**
(43) Date of publication of application: **08.10.2025**
(21) Application number: 24168466.1
(22) Date of filing: 04.04.2024
(51) Int. Cl.: A61B 6/00, A61B 6/03, F28D 20/02, H05G 1/02, A61B 6/58, F24D 15/02

(54) **THERMAL BUFFER AND MEDICAL DEVICE WITH THERMAL BUFFER**

(71) Applicant: Koninklijke Philips N.V., 5656 AG Eindhoven (NL)
(72) Inventor: VOGTMEIER, Gereon, Eindhoven (NL); WEIß, Steffen, Eindhoven (NL); JOHNSON, Mark Thomas, 5656AG Eindhoven (NL); LEUSSLER, Christoph Günther, Eindhoven (NL); LIPS, Oliver, Eindhoven (NL)
(74) Representative: Philips Intellectual Property & Standards

(57) **Abstract**

The present disclosure is directed to a thermal buffer. A thermal buffer for thermal impact on at least a part of a device is provided, wherein the buffer comprises a buffer body having a thermal storage capacity of at least 120 kJ/kg and a thermal conductivity of at least 0.15 W/(m•K), wherein the buffer is configured to be detachably attached to at least a part of the device, optionally to an outer surface of the device. The buffer body comprises an interface for direct or indirect thermal contact between at least a part of the buffer body and at least a part of the device, so that the buffer is configured for direct or indirect heat conduction between the device and the buffer body via the interface, when the buffer is attached to at least a part of the device, optionally for and/or during operation of the device.

## Description

### FIELD OF THE INVENTION

The present invention refers to a thermal buffer, in particular for mobile medical devices and/or mobile medical imaging devices, a medical device comprising such thermal buffer, the use of a thermal buffer, a thermal modification apparatus for heating and/or cooling of the thermal buffer, and a control unit.

### BACKGROUND OF THE INVENTION

For devices, such as medical devices, more specifically mobile medical devices and/or medical imaging devices, e.g. x-ray tubes, but also for other imaging systems, a simple but sufficient cooling at defined locations is necessary. More generally, it is desired to improve thermal conditioning, in particular cooling, with respect to such devices, in particular in terms of costs and/or handling and/or reliability.

### SUMMARY OF THE INVENTION

The present invention is directed to the thermal buffer of claim 1. Accordingly, a thermal (i.e. cooling and/or heating) buffer for providing thermal impact (i.e. thermal conditioning - cooling and/or heating) on at least a part of a device is provided, wherein the buffer comprises a buffer body having a thermal storage capacity of optionally at least 120 kJ/kg (between a lower and an upper temperature of the buffer during thermal conditioning, i.e. cooling and/or heating, of the device) and/or a thermal conductivity of optionally at least 0.15 W/(m·K) (between a lower and an upper temperature of the buffer during thermal conditioning, i.e. cooling and/or heating, of the device), wherein the buffer is configured to be (detachably) attached to at least a part of the device, optionally to an outer surface (e.g. a part of a housing) of the device. The buffer body comprises an interface for direct or indirect thermal contact between at least a part of the buffer body and at least a part of the device, so that the buffer is configured for direct or indirect heat conduction (transfer of heat) between the device and the buffer body via the interface, when the buffer is attached to at least a part of the device, optionally for and/or during operation of the device.

A thermal/temperature buffer may be a cooling and/or heating buffer. The buffer may be referred to as a thermal energy storage/pack, in particular for thermal influence of the buffer on the device. Hence, the buffer body may absorb heat from the device, thus acting as a cooling buffer; or may transfer heat to the device, thus acting as a heating buffer.

While thermal impact/conditioning refers to both cooling or heating, an optional and preferred embodiment refers to cooling (not heating).

The buffer may be regarded as a passive buffer in that it provides passive thermal impact on the device. In particular, the buffer may passively cool/heat the device. The buffer itself may not need to be actively cooled/heated during use of the buffer for cooling/heating the device. The buffer may be configured to be free of active cooling/heating means. For example, the buffer may be free of (electric and/or fluid) connections to a stationary apparatus, which may be necessary for active cooling/heating of the buffer.

The buffer may be removable/replaceable, i.e. the buffer may be removed from the device. For example, the buffer may be a portable/mobile buffer.

The buffer may be an external buffer for provision/attachment to an outside of the device, e.g. external to the device.

Thermal storage capacity may refer to (latent) heat storage in the buffer.

For example, a lower (operating) temperature may be the temperature at which the device starts to operate (typically identical to ambient temperature); and an upper (operating) temperature may be a lower or higher temperature of the device, e.g. a higher temperature from which on the device needs to be cooled by the buffer.

The specific melting heat of suitable materials of the buffer body may optionally be in the range of 150-250 J/g, e.g. as for the following materials: paraffm/metal mixtures, paraffin polymer composites, paraffin carbon mixtures, pure paraffin. These materials offer about half the cooling capacity of water (melting heat of 334 J/g which is exceptionally high) but they still can be stored "loaded" at normal ambient temperatures, unlike water ice, because the phase change temperature of these materials is above normal ambient temperatures.

The buffer is in particular configured for mobile medical devices free of active cooling (or heating) means, but also configured as an add-on (supplementary means) for stationary (medical, e.g. imaging) devices, in particular those already having an active cooling (or heating) means. For example, the thermal buffer may allow devices to be devoid of (other) cooling/heating means, in particular of active cooling/heating means or may provide a back-up cooling/heating means.

The thermal buffer may be used for pre-conditioning the device, before operation of the device, to a desired temperature.

The thermal buffer of the invention may provide simplification of the heat management system of a device, such as of a medical (diagnostic) device, more specifically a mobile medical device, such as a mobile imaging system. Specifically, the device may be an electric device and/or may comprise a heat source.

By way of the buffer, reliable cooling/heating may be possible even in remote areas (where no active cooling/heating is possible) or in case of interrupted/broken power supply. Also, the buffer allows for cost effective realization of cooling/heating. In particular, the buffer may be of a relatively simple configuration, versatile and re-usable. Further, the buffer may allow for convenient handling, as e.g. the buffer may have a relatively low weight and/or may have reasonable dimensions. Fast replacement/exchange of buffers, e.g. pre-cooled cooling packs, may be even faster and more efficient compared to battery-buffered systems. The buffer may allow for cooling/heating of the device from its outside, so that positioning thereof at the device may not be intricate. Different geometries of the buffer may allow for a flexible and modular concept and/or for customization, e.g. depending on environmental conditions, such as in different regions of the world without re-designs or extra costs.

Specifically, a thermal storage capacity of at least 120 kJ/kg may support the provision of sufficient cooling/heating energy, and a thermal conductivity of at least 0.15W/(m-K) may support an appropriate timing for the heat exchange. Together, these parameters may specifically support reliable and sufficient energy exchange between the buffer body and at least a part of the device.

Optionally, the buffer comprises attachment means for attachment to the device and/or for connection to another part of the buffer. Further optionally, the attachment means may allow for connection to other body portions of buffer, if the buffer comprises more than one buffer portion. Attachment means may help to position and locate the buffer at an appropriate part of the device.

Optionally, the shape/geometry of at least a part of the buffer in at least one state is configured to conform to at least a part of the device, in particular its outer surface. For example, the buffer, e.g. the buffer body, may have a complementary shape which is complementary to the part of the device where the buffer is to be located

Optionally, the interface comprises an area made of thermally conductive polymers and/or composite materials and/or metal, optionally copper and/or carbon. This may swiftly and reliably support heat transfer between the buffer body and the device.

Optionally, the buffer body at least in parts comprises a phase change material.

This means that the buffer body undergoes a phase change (between a lower and an upper temperature of the buffer). Phase-change material (PCM) may be referred to as latent heat storage (LHS) materials. This may allow for efficient cooling, as the latent heat is made use of.

Optionally, the phase change material may have a melting point above normal ambient temperature, e.g. between 15 to 25 °C. In this case, cooling, i.e. re-charging, of the material of the buffer body may be simply done by heat exchange with ambient air. In this case, no active cooling of the buffer in a docking station after use may be necessary.

If the buffer comprises a phase change material, materials based on mixtures of paraffin waxes with high viscosity materials to maintain shape stability in the liquid phase and also with highly thermally conductive materials may allow for efficient heat transfer.

Optionally, the shape/geometry of the buffer body in the liquid phase basically corresponds to the shape/geometry of the buffer body in the solid phase. As such, it is preferable that a phase change of the buffer body does not mean a change of the geometry of the buffer body. This may particularly simplify handling of phase change materials. An example material is paraffin.

Optionally, the buffer has a sensor for determination of the temperature of the buffer body. The sensor may be a temperature sensor. By way of the indication of the temperature of the buffer body, an indication of the buffer capacity can be obtained. For example, during cooling of a device by means of the buffer, the remaining cooling capacity of the buffer may be detected, e.g. monitored.

If temperature sensors or optical sensors are present in or near the buffer, the buffer may be configured to measure the state of the buffer (temperature, liquid/solid) and may thus declare a buffer as "usable" or "still regenerating/recharging".

Optionally, the buffer body comprises at least first and second body portions. In other words, the buffer body may comprise multiple portions. The portions may be individual portions, provided separately from each other. Alternatively, the portions may be in contact with each other, e.g. connected to each other. This may allow for contacting different parts of the device by different buffer portions, e.g. different thermal sources of the device. Alternatively or additionally, combining (e.g. stacking) buffer portions to increase the overall capacity of the buffer body may be desired. As such, multiple buffer body portions may allow for modular and flexible forming of the buffer body.

The invention is also directed to a device, optionally a medical device, more optionally a mobile and/or imaging device. The device is configured for detachable attachment of a thermal buffer, optionally the thermal buffer of the invention, to at least a contact area of the device, optionally a part of an outer surface (e.g. of the housing) of the device, wherein the contact area of the device is configured for direct or indirect heat exchange between the buffer body and at least a part of the device.

The device may not have a continuous/active cooling/heating, but it may be sufficient to provide thermal conditioning via the (passive) buffer. More specifically, the device may be free of a (fluid and/or electric) cooling/heating circuit for cooling/heating at least a part of the medical device. This may help to reduce costs for the device as such, as no active, more intricate thermal conditioning means for the device may be needed. Alternatively, the buffer may provide supplementary cooling/heating means, in addition to active cooling/heating means of the device. The mobile device may, thus, be less complex, more lightweight and may allow for simpler maintenance.

The device may be configured such that the buffer is not in contact with internal parts of the device. For example, in case of refrigerant in the device, the buffer may not be in contact with refrigerant.

The contact area may be in direct or indirect contact with the thermal source, e.g. heat source. An indirect thermal contact may include heat transfer via a heat guiding element, such as a heat pipe, e.g. for CPU in PC systems. As such, a heat source of the device may be connected to the contact surface so as to direct the heat generated elsewhere in the device towards the contact area of the device for transmission to the interface and, hence, absorption by the buffer.

Optionally, the contact area at least in part is made of thermally conductive polymers and/or composite materials and/or metal, optionally copper and/or carbon, for thermal conduction with the interface of the buffer body. This may offer swift and reliable support for heat transfer between the buffer body and the device.

The invention is also directed to the use of a thermal buffer, optionally of the invention, at a device, optionally the device of the invention, wherein the use comprises attaching the thermal buffer to the device, optionally to at least a part of an outer surface (housing) of the device.

The invention is also directed to a thermal modification (thermally re-charging) apparatus for cooling and/or heating of at least a body of a thermal buffer, optionally the buffer of the invention, for heating and/or cooling of a device, wherein the apparatus comprises at least a cooling and/or heating unit configured to cool and/or heat at least the body of the thermal buffer and further a receptacle for at least the body, when at least the body is being cooled and/or heated (in the thermal modification apparatus), and further comprises a cooling and/or heating control unit configured to control the cooling and/or heating unit based on a thermal requirement for a targeted operation of the device, and to receive information on the temperature of the body of the thermal buffer, when the body is being cooled and/or heated (in the thermal modification apparatus).

This may in particular allow to re-use the buffer. Also, it may allow for pre-determined and appropriate temperature adjustment of the buffer. The thermal modification apparatus may be a cooling and/or heating apparatus and/or may be seen as a docking/storage station for the buffer, in particular for storage and/or re-charging of the buffer.

Optionally, the thermal modification apparatus comprises an optical sensor for determining a state of the body of the buffer, in particular whether the body is in a liquid or solid state, and/or a temperature sensor for determining the temperature of the body of the buffer. This may allow for monitoring of the buffer, e.g. visually and/or electronically.

The invention is also directed to a method and control unit, optionally in a device or in a thermal modification apparatus of the invention, configured to carry out the following steps: obtaining information regarding a thermal buffer, optionally a buffer of the invention, in particular regarding its thermal storage capacity and its current temperature, optionally by way of a sensor for determining the temperature of a body of the thermal buffer, including determining an available heat storage amount of the thermal buffer, when the buffer is attached to the device for direct or indirect heat conduction between the device and the buffer body, andobtaining information regarding operational parameters of the device for an ongoing or a targeted operation of the device, and deriving, on the basis of the available heat storage amount and the operational parameters, an indication pertaining to the use of the thermal buffer for the targeted operation.

The steps may relate to the buffer during recharging, i.e. when received in the thermal modification apparatus, and to a planned use/operation of the device. Alternatively, the steps may relate to the buffer during use, i.e. when the device is being used, and the remaining thermal capacity of the buffer in view of the operation of the device.

For example, steps pertaining to the thermal buffer may include the selection of the buffer size (e.g. which body or how many portions/layers in case of a stack of buffer elements).

Information pertaining to the device may be when the system has to be prepared with the expected temperature and be ready for use and/or to optimized pre-cooling scenario knowing the later operation condition of the device (scan-time, kV, mA, environmental temperature at system, delay times, waiting time, transportation time to operation). This may help to improve efficiency for the thermal modification during use. The control (method or unit) may be configured to monitor if the device is being used as planned and to otherwise alert the user (practitioner, clinician). These steps may also relate to the cooling and/or heating control unit of the thermal modification apparatus. In particular, the cooling and/or heating control unit may be the control unit of the invention.

Optionally, the method or control unit is configured to derive an indication of an appropriate thermal pre-conditioning of the thermal buffer for a targeted operation and/or configured to derive a remaining time period for an ongoing operation of the medical device based on the remaining available thermal storage capacity.

While embodiments according to the figures relate to medical devices, the buffer of the disclosure is not limited to an application to medical and/or mobile devices, but may be configured for use with any suitable device. Also, devices of the disclosure are not limited to medical and/or mobile devices.

### BRIEF DESCRIPTION OF THE DRAWINGS

Fig. 1 shows a schematic perspective view of a buffer and a medical device according to embodiments of the invention.
Fig. 2 shows a schematic perspective view of another buffer and another medical device according to embodiments of the invention.
Fig. 3 shows a schematic perspective view of another buffer and another medical device according to embodiments of the invention.
Fig. 4 shows a schematic top view of a buffer of an embodiment of the invention.
Fig. 5 shows a schematic side view of a buffer of an embodiment of the invention.
Fig. 6 shows a schematic top view of a thermal modification apparatus of an embodiment of the invention.

### DETAILED DESCRIPTION OF EMBODIMENTS

A thermal buffer 1 provides thermal impact on at least a part of a device 2. The buffer 1 is configured for direct or indirect heat conduction between the device 2 and the buffer body 3 via the interface 5 of the buffer 1, when the buffer 1 is attached to at least a part of the device 2, namely the contact area 7 of the device 2, in particular during operation of the device 2. The buffer 1 may allow for appropriate passive temperature-management during operation of the device 2, thus e.g. allowing for stable conditions of the device, e.g. imaging conditions of an imaging device. This may be achieved by installing buffer(s) 1 at critical locations, such as the contact area 7. A workflow as preconditioning may be done based on the planned imaging procedure (e.g. number of x-ray scans and the correlated generated heat that has to be "compensated" by the preconditioned heat buffer 1).

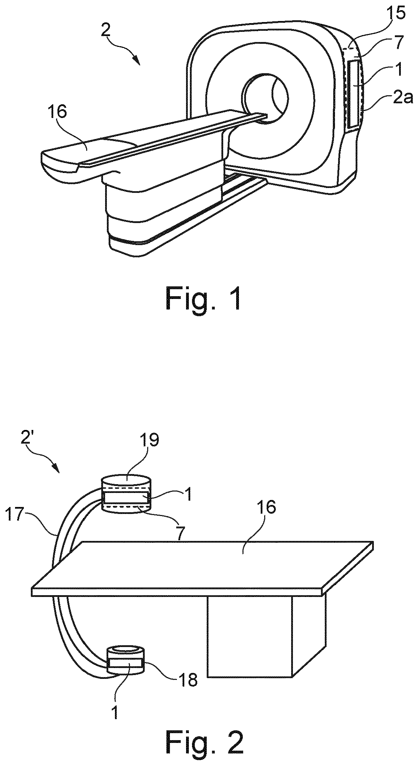

Fig. 1 shows the medical device 2, e.g. a CT or MRI imaging apparatus, configured for detachable attachment of the thermal buffer 1 to at least a contact area 7 of the medical device 2, e.g. a part of an outer surface 2a of the medical device 2, wherein the contact area 7 of the medical device 2 is configured for heat exchange between the buffer body 2 and at least a part of the medical device 2. The contact area 7 is at least in parts made of thermally conductive polymers and/or composite materials and/or metal, optionally copper and/or carbon, for thermal conduction with the interface 5 of the buffer body 3. Fig. 1 further shows the patient table 16 and the gantry 15, at which the attachment area 7 is located. The buffer 1 is detachably attached to at least a part of the device 2, here to an outer surface 2a of a housing of the device 2.

Fig. 2 show another medical device 2' comprising a C-arm 17, i.e. an imaging apparatus having an X-ray source 19 and a detector 18 positioned at opposite ends of the arm. At each of the X-ray source 19 and the detector 18, a buffer 1 is located. The buffer 1 cools the X-ray source 19 during use, when heat is generated in the anode (not shown). The buffer 1 at the detector 18 may be for cooling of the detector 18 during use. Another buffer 1 may be used for heating of the detector 18 before use, e.g. to bring the detector 18 to a constant temperature during use. The medical device 2' further comprises a table 16 for positioning of a patient.

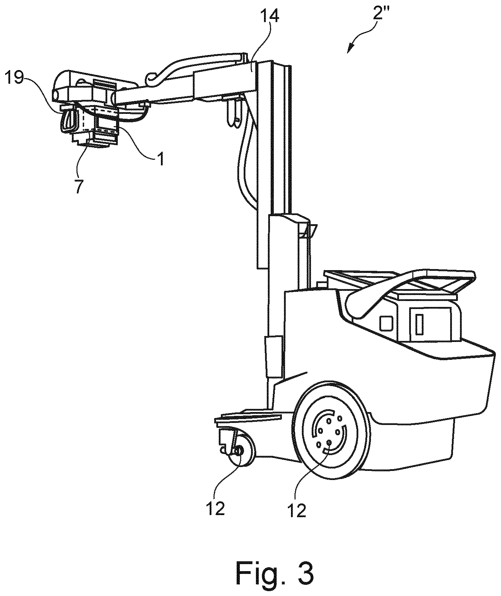

Fig. 3 shows a mobile diagnostic X-ray imaging device 2", having wheels 12 and a mechanical beam (arm) 14 supporting the X-ray source 19. The housing of the device 2" comprises the contact area 7 for attachment of the buffer 1. In Fig. 3, such buffer 1 is attached to the device 2".

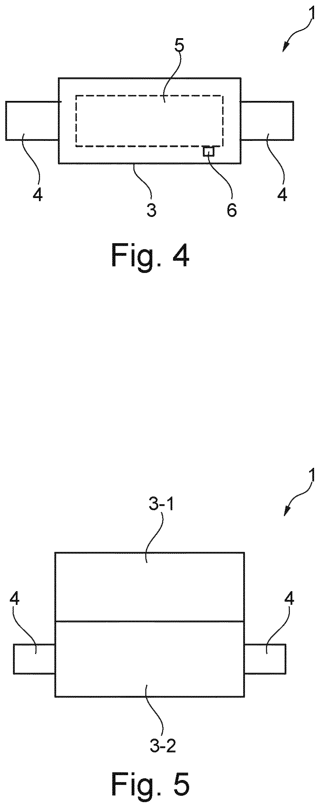

As shown in Fig. 4 in more detail, the buffer 1 comprises a buffer body 3 having a thermal storage capacity of at least 120 kJ/kg between a lower and an upper temperature of the buffer 1 and a thermal conductivity of at least 0.15W/(m-K).

The body 3 may be in a slot cassette or in thermal contact with a heat guiding element, e.g. a heat pipe (not shown). By way of the heat guiding element, the thermal energy of a distanced part of the medical device 2, 2', 2" may be in (indirect) thermal contact with the contact area 7 of the medical device. The buffer body 2 comprises an interface 5 for direct or indirect thermal contact between at least a part of the buffer body 3 and at least a part of the device 2, namely the contact area 7 of the device 2. The buffer 1 further comprises attachment means 4 for attachment to the device 4, in particular to its contact area 7, and/or for connection to another part of the buffer 1. The shape/geometry of at least a part of the buffer 1 in at least one state is configured to conform to at least a part of the device 2.

The interface 5 comprises an area made of thermally conductive polymers and/or composite materials and/or metal, optionally copper and/or carbon. Hence, the buffer 1 is equipped with a mechanical heat sink, namely the interface 5.

The buffer body 3 at least in parts comprises a phase change material. A shape/geometry of the buffer body 3 in the liquid phase basically corresponds to the shape/geometry of the buffer body 3 in the solid phase. An advantage of passive cooling with phase change cooling packs with melting temperature above normal ambient temperature is that many of these packs 3 can be distributed in even small portions at structures critical to be cooled, because they do not need to be isolated (to keep cool) nor is any access to them required because they do not need to be actively "reloaded" by external cooling. They rather simply regenerate themselves after usage of the device 2, 2', 2" to be cooled when they cool down to normal ambient temperature. Examples to place many of such buffer bodies 3 are e.g. directly at the housing 2a close to the bearing of a mobile X-ray tube 19, at all places that receive X-ray and thermal radiation, such as the detector 18, at the HV generator, and at critical electronics. For example, the anode (not shown) may be a heat-generating, i.e. hot, element inside the X-ray tube 19, wherein heat is transferred from the anode to the housing 2a. More specifically, a part of the heat may be radiated to the housing 2a and a part may be transported via the rotation axis and the bearing to the housing 2a. At least a part of the housing 2a forms the contact area 7. Alternatively or additionally, the detector 18 may be a heat-generating, i.e. hot, element. More specifically, readout electronics may be heating up the detector 18. As scintillator material (for conversion of X-rays into photons), however, is temperature sensitive and also a photodiode, it is preferable in terms of performance to keep the temperature of the detector 18 stable and low.

In the medical devices 2, 2', 2", the connection of e.g. the heat buffer 1, e.g. based on phase transition materials, to the heat source of the mobile device is ensured via the contact between the interface 5 of the buffer and the contact area 7 of the medical device, so that energy transfer e.g. from a heat generation source (in the medical device 2, 2', 2") to the buffer 1 is established.

Suitable phase change materials are materials based on mixtures of paraffin waxes with high viscosity materials to maintain shape stability in the liquid phase and also with highly thermally conductive materials to allow efficient heat transfer. The phase change temperature of these materials (typically 45°C to 65°C) is chosen per chemical design to be still in the solid phase at the highest expected environmental ambient temperature. This means that buffers 1 may be stored in the "loaded" state without any active cooling or isolation. At the same time, these materials maintain their outer shape also in the liquid phase, which simplifies integration of the buffer 1 in the device 2, 2', 2" to be cooled, and maintains thermal contact between buffer 1 and device 2, 2', 2" to be cooled.

As shown in Fig 4, the buffer 1 has a sensor 6, in particular at the buffer body 3, for determination of the temperature of the buffer body 3.

Fig. 5 shows that the buffer body 3 comprises at least first 3-1 and second 3-2 body portions. The body portions may be stacked, i.e. on top of each other, and/or may be located next to each other. The multiple body portions 3-1, 3-2 may each comprise attachment means 4 or may share common attachment means 4. Different "add-on packs" 3-1, 3-2 depend on the typical environmental condition where the buffer 1 is used. In that way, an optimized configuration may be achieved with the benefit of low weight.

The buffer 1 is connected to the source in the medical device 2, 2', 2" via the mechanical interface 5 to optimally transfer heat to the buffer 1. A flat or interdigital 3D surface connection to optimally guarantee the heat transfer is possible. A tight and reliable fitting is preferable, which may be controlled by sensing devices (not shown). Fitting may be realised by passive or electromechanical actuator device (not shown).

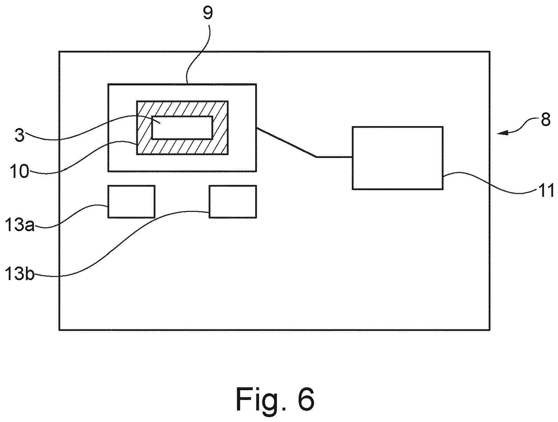

Fig. 6 shows a thermal modification apparatus 8 for cooling and/or heating of at least a body 3 of a thermal buffer 1, wherein the apparatus 8 comprises at least a cooling and/or heating unit 9 configured to cool and/or heat at least the body 3 of the thermal buffer 1 and a receptacle 10 for receiving at least the body 3, when at least the body 3 is being cooled and/or heated. The cooling and/or heating unit 9 may comprise the functionality of cooling down/heating up and is integrated in the apparatus, i.e. storage box/docking station 8. The apparatus 8 further comprises a cooling and/or heating control unit 11 configured to control the cooling and/or heating unit 9 based on a thermal requirement for a targeted operation of the device 2, 2', 2" and to receive information on the temperature of the body 3 of the thermal buffer, when the body 3 is being cooled and/or heated by the apparatus 8.

The thermal modification apparatus 8 comprises an optical sensor 13b for determining a state of the body 3 of the buffer, in particular whether the body 3 is in a liquid or solid state. The apparatus 8 of Fig. 6 further comprises a temperature sensor 13a for determining the temperature of the body 3 of the buffer 1. Alternatively or additionally, the temperature sensor 6 of the buffer may determine the temperature of the body 3.

For example, liquid cooling may be used to transfer energy from/to the passive buffer 1 for thermal re-charging in the cooling and/or heating unit 9. This may be seen as active "re-charging" of the buffer 1 by liquid cooling in the unit 9. The buffer 1 may, for example, be equipped with a liquid cooling method to transfer and to distribute the heat into the buffer 1. This allows to optimally transfer peaks of energy into the buffer 1.

The buffer 1 may be used for a mobile medical system 2, 2', 2", including heat generating components in operation, wherein the components are connected to the passive heat buffer 1 that is configured to be pre-cooled when connected to a docking station (storage box), i.e. thermal modification apparatus 8, and that takes the heat energy from the heat generating components, such as the X-ray source 19, e.g. during the few shots of mobile operation.

The cooling and/or heating control unit 11 may be provided in the medical device 2, 2', 2" or in the thermal modification apparatus 8, as shown in Fig. 6. The unit 11 is configured to carry out the following steps: obtaining information regarding the thermal buffer 1, in particular regarding its thermal storage capacity and its current temperature, optionally by way of a sensor 6 for determining the temperature of a body 3 of the thermal buffer, including determining an available heat storage amount of the thermal buffer, when the buffer 1 is attached to the medical device 2, 2', 2" for direct or indirect heat conduction between the medical device and the buffer body, and obtaining information regarding operational parameters of the medical device 2, 2', 2" for an ongoing or a targeted operation of the medical device, and deriving, on the basis of the available heat storage amount and the operational parameters, an indication pertaining to the use of the thermal buffer 1 for the targeted operation.

The control unit 11 is configured to derive an indication of an appropriate thermal pre-conditioning of the thermal buffer 1 for a targeted operation and/or configured to derive a remaining time period for an ongoing operation of the medical device 2, 2', 2" based on the remaining available thermal storage capacity. In other words, in some embodiments, pre-cooling of the buffer 1 to a desired temperature may allow for safe (X-ray tube 19) operation during the planned scans. The scheduling information as one input parameter may adapt the conditioning and also the operation may be controlled by the unit 11 according to the heat capacity and the pre-cooling.

The temperature monitoring may be done (in a passive mode for the buffer 1) in the apparatus 8 and/or in the medical device 2, 2', 2" (e.g. by means of a temperature sensor 13a in the apparatus 8 and/or the medical device 2, 2', 2") or (in an active mode with) in the temperature sensor 6 in the buffer body 3. Optionally, communication means (not shown) for communication with the medical device 2, 2', 2" to inform about the buffer status and/or with the apparatus 8 to monitor the pre-conditioning of the buffer 1 may be provided. It is also conceivable that the apparatus 8 and the medical device 2, 2', 2" are in communication with each other by means of the communication means. E.g. upcoming requests for cooling including the temperature request (depending on future imaging plans) may be exchanged. Alternatively or additionally, warnings/alerts in case of a deviation from a plan/schedule and/or out of range temperature info may be handled in this way.

For example, steps pertaining to the thermal buffer 1 may include the selection of the buffer size (e.g. which body or how many portions/layers 3-1, 3-2 in case of stack of buffer elements). Information pertaining to the device 2, 2', 2" may be when the device has to be prepared with the expected temperature and be ready for use and/or to optimized pre-cooling scenario knowing the later operation condition of the device (e.g. scan-time, kV, mA, environmental temperature at system, delay times, waiting time, transportation time to operation). This may help to improve efficiency for the thermal modification during use. The control (method or unit 11) may be configured to monitor if the device 2, 2', 2" is being used as planned and to otherwise alert the user (practitioner, clinician). These steps may relate to the cooling and/or heating control unit 11 of the thermal modification apparatus 8. In particular, the cooling and/or heating control unit 11 may be the control unit of the invention.

The method/control unit 11 may initiate a medical workflow such that at a desired/needed point in time the medical device 2, 2', 2" is ready for use and the status of the pre-conditioned medical device 2, 2', 2" is that it is able to carry out the requested operation - e.g. the number of x-ray shots / scans. With the scan-parameters (e.g. kV, mA and time per scan), the required cooling for the expected cooling of the X-ray tube 19 may be determined. As such, it may be possible to appropriately prepare the buffer 1 for a planned operation mode/time. A controller/user may assist so that the device is ready for use at the planned time of usage.

The thermal modification apparatus 8 and/or the control unit 11 may be equipped with temperature sensors 13a or optical sensors 13b, which measure the state of the buffer 1 (temperature, liquid/solid) and may thus declare a buffer 1 as "usable" or "still regenerating". Moreover, also the buffer 1 may be equipped with sensors 6 to determine the temperature and the remaining "capacity", such that warnings or instructions to replace the buffer 1 may be generated, e.g. by the thermal modification apparatus 8 and/or the medical device 2, 2', 2".

### LIST OF REFERENCE SIGNS:

- 1: Thermal buffer
- 2, 2', 2": Medical device
- 2a: Outer surface (housing) of medical device
- 3: Buffer body
- 3-1, 3-2: First and second buffer body portions
- 4: Attachment means
- 5: Interface
- 6: Sensor of buffer
- 7: Contact area
- 8: Thermal modification apparatus
- 9: Cooling and/or heating unit
- 10: Receptacle
- 11: Cooling and/or heating control unit
- 12: Wheels
- 13a: Temperature sensor
- 13b: Optical sensor
- 14: Arm
- 15: Gantry
- 16: Patient table
- 17: C-arm
- 18: Detector
- 19: X-Ray tube

## Claims

1. Thermal buffer (1) for providing thermal impact on at least a part of a device (2, 2', 2"),
the buffer (1) comprising a buffer body (3) having a thermal storage capacity of at least 120 kJ/kg and a thermal conductivity of at least 0.15W/(m-K),
the buffer body (2) comprising an interface (5) for direct or indirect thermal contact between at least a part of the buffer body (3) and at least a part of a contact area (7) of the device (2, 2', 2"),
the buffer (1) configured to be detachably attached to at least a part of the contact area (7) of the device, optionally of an outer surface (2a) of the device,
so that the buffer (1) is configured for direct or indirect heat conduction between the device (2, 2', 2") and the buffer body (3) via the interface (5), when the buffer is attached to at least a part of the contact area (7) of the device (2, 2', 2"), optionally during operation of the device.

2. Thermal buffer of claim 1, wherein the buffer (1) comprises attachment means (4) for attachment to the device (4) and/or for connection to another part of the buffer (1).

3. Thermal buffer of claim 1 or 2, wherein the shape/geometry of at least a part of the buffer (1) in at least one state is configured to conform to at least a part of the device (4), optionally the part including the contact area (7).

4. Thermal buffer of any of the preceding claims, wherein the interface (5) comprises an area made of thermally conductive polymers and/or composite materials and/or metal, optionally copper and/or carbon.

5. Thermal buffer of any of the preceding claims, wherein the buffer body (3) at least in parts comprises a phase change material.

6. Thermal buffer of any of the preceding claims, wherein a shape/geometry of the buffer body (3) in the liquid phase basically corresponds to the shape/geometry of the buffer body (3) in the solid phase.

7. Thermal buffer of any of the preceding claims, wherein the buffer (1) has a sensor (6) for determination of the temperature of the buffer body (3).

8. Thermal buffer of any of the preceding claims, wherein the buffer body (3) comprises at least first (3-1) and second (3-2) body portions.

9. Medical device (2, 2', 2"), optionally mobile medical device and/or medical imaging device, configured for detachable attachment of a thermal buffer (1), optionally the thermal buffer of any of the preceding claims, to at least a contact area (7) of the medical device, optionally of an outer surface (2a) of the medical device,
wherein the contact area (7) of the medical device (2, 2', 2") is configured for heat exchange between the buffer body (2) and at least a part of the medical device (2, 2', 2").

10. Medical device of claim 9, wherein the contact area (7) is at least in parts made of thermally conductive polymers and/or composite materials and/or metal, optionally copper and/or carbon, for thermal conduction with the interface (5) of the buffer body (3).

11. Use of a thermal buffer (1), optionally of any of the preceding claims 1 to 8, at a medical device (2, 2', 2"), optionally of any of the preceding claims 9 and 10, wherein the use comprises attaching the thermal buffer (1) to the medical device (2, 2', 2"), optionally to at least a part of an outer surface (2a) of the medical device.

12. Thermal modification apparatus (8) for cooling and/or heating of at least a body (3) of a thermal buffer (1), optionally the buffer (1) of any of the preceding claims 1 to 8, for heating and/or cooling of a device (2, 2', 2"), wherein the apparatus (8) comprises at least
a cooling and/or heating unit (9) configured to cool and/or heat at least the body (3) of the thermal buffer (1) and comprising a receptacle (10) for at least the body (3), when at least the body (3) is being cooled and/or heated,
a cooling and/or heating control unit (11) configured to control the cooling and/or heating unit (9) based on a thermal requirement for a targeted operation of the device (2), and to receive information on the temperature of the body (3) of the thermal buffer, when the body (3) is being cooled and/or heated.

13. Thermal modification apparatus of claim 12, further comprising an optical sensor (13b) for determining a state of the body (3) of the buffer, in particular whether the body is in a liquid or solid state, and/or a temperature sensor (13a) for determining the temperature of the body (3) of the buffer (1).

14. Control unit, optionally in a medical device (2) or in a thermal modification apparatus (8), more optionally of any of the preceding claims, configured to carry out the following steps:
obtaining information regarding a thermal buffer (1), in particular regarding its thermal storage capacity and its current temperature, optionally by way of a sensor (6) for determining the temperature of a body (3) of the thermal buffer, including determining an available heat storage amount of the thermal buffer, when the buffer (1) is attached to the medical device (2) for direct or indirect heat conduction between the medical device and the buffer body (3), and
obtaining information regarding operational parameters of the medical device (2, 2', 2") for an ongoing or a targeted operation of the medical device, and
deriving, on the basis of the available heat storage amount and the operational parameters, an indication pertaining to the use of the thermal buffer (1) for the targeted operation.

15. Control unit of claim 14, configured to derive an indication of an appropriate thermal pre-conditioning of the thermal buffer (1) for a targeted operation and/or configured to derive a remaining time period for an ongoing operation of the medical device (2) based on the remaining available thermal storage capacity.
